Europäisches Patentamt

European Patent Office

Office européen des brevets

(11) Publication number: **0 308 218 B1**

# EUROPEAN PATENT SPECIFICATION

(45) Date of publication of patent specification: **26.02.92** (51) Int. Cl.⁵: **A61F 5/448**

(21) Application number: **88308529.2**

(22) Date of filing: **15.09.88**

(54) **Bayonet coupling for ostomy device.**

(30) Priority: **16.09.87 US 96989**

(43) Date of publication of application:
**22.03.89 Bulletin 89/12**

(45) Publication of the grant of the patent:
**26.02.92 Bulletin 92/09**

(84) Designated Contracting States:
**AT BE CH DE ES FR GB GR IT LI LU NL SE**

(56) References cited:
**EP-A- 0 163 979**
**DE-A- 3 637 355**
**GB-A- 27 254**
**GB-A- 2 193 893**

(73) Proprietor: **E.R. Squibb & Sons, Inc.**
**Lawrenceville-Princeton Road**
**Princeton, N.J. 08540-4000(US)**

(72) Inventor: **Leise, Walter Francis**
**19 South Homestead Drive**
**Yardley Pennsylvania(US)**
Inventor: **Johnsen, Kenneth Alan**
**54 Seward Avenue**
**Piscataway New Jersey(US)**

(74) Representative: **Cook, Anthony John et al**
**D. YOUNG & CO. 10, Staple Inn**
**London, WC1V 7RD(GB)**

EP 0 308 218 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to body waste receptacles and more particularly concerns a coupling device for use in connecting an ostomy bag to a wearer.

Ostomy couplings are known. These employing a bayonet-type mechanism to attach the two coupling parts together are exemplified by the following patent documents: US-A-3736934 (on which the preamble of claim 1 is based), DE-A1-3527897, BE-A-853876, and GB-A-27254 of 1909. Some couplings have employed O-rings or equivalent seals for sealing purposes. Examples are: DE-A-3637355, US-A-3398744 and GB-A-2193893. This latter document was published after the present Applicant's priority date.

The aim of the present invention is to provide a particularly effective construction in which access of faecal solids to the area adjacent the O-ring is substantially prevented.

According to the invention, there is provided a coupling coupling device for use in connecting an ostomy bag to a wearer of the type comprising a body side coupling flange having a substantially circular shape with an aperture therein adapted to surround a stomal opening in a wearer and a flat surface thereon adapted to interface with an adhesive material for connecting said body side coupling to said wearer, and a bag side coupling flange adapted to be connected to said body side coupling, said bag side coupling holding a bag for receiving material which passes from said wearer, characterised by the combination of a bayonet coupling with an O-ring seal, said bayonet coupling connecting said body side flange to said bag side flange without requiring significant pressure to be applied against said body side flange; the O-ring seal providing a seal between said body side flange and said bag side flange, and being comprised of an O-ring which is resiliently held within a radially outwardly opening annular groove formed in an annular wall of the body side flange; said body side flange further including a plurality of radially extending pins and said bag side flange including a first annular wall having a like number of openings for receiving said pins, said openings having an L-shaped configuration whereby said bag side flange and body side flange can be joined and held together; the bag side flange also having a second annular wall located radially inwardly of the first annular wall, said further annular wall having an outer surface which contacts an inner surface of the body side flange annular wall to prevent solids from entering the seal area adjacent to the O-ring.

The bayonet flange system disclosed herein provides an easy to use means of coupling an ostomy bag and wafer. The sealing and locking occur at different places. This allows coupling and uncoupling forces to remain very low, making it an ideal appliance to use post surgically to limit trauma at the surgical site. The flanges are coupled to one another by placing the arm of the bag side flange over the arm of the body side flange and twisting in a clockwise motion with a slight downward pressure. The body side pins will mate with the bag side slots and then move into the locked position. Because of its easy operation and reliable sealing and locking, this concept may also be used by ostomates on a regular basis.

In the accompanying drawings:-

Figure 1 is a cross-sectional view of the bayonet coupling system of the present invention;

Figure 2 is a bottom view of said body side coupling element;

Figure 3 is a top view of said bag side coupling element; and

Figure 4 is a side view of the bag side coupling element of Figure 3 taken along the lines 44 in Figure 3.

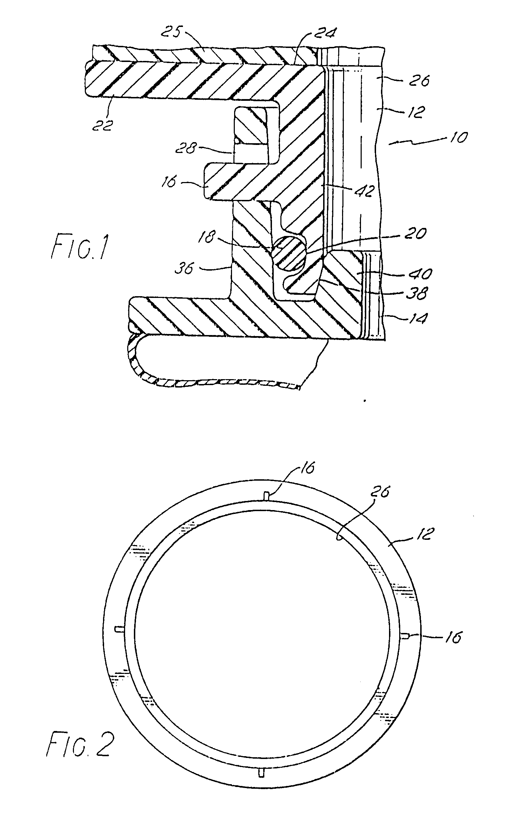

Referring generally to Figure 1, the bayonet flange system 10 of the present invention is comprised of a body side flange 12 and a bag side flange 14 designed to interlock with one another. The body side flange 12 has a plurality of locking pins 16 and a separate seal which is an O-ring seal 18 in the preferred embodiment of the invention 10. The O-ring seal 18 is held on the body side flange 12 by its internal tension and is located in an O-ring groove 20 formed within the body side flange 12. The body side flange 12 includes a leg 22 which provides a large flat surface 24 which may be used to bond the flange 12 to a wafer 25 of an adhesive, such as Stomahesive used to bond the flange 12 to a wearer's body. The body side flange 12 is substantially circular in shape and includes a central aperture 26 which surrounds a wearer's stomal opening when the invention 10 is in use.

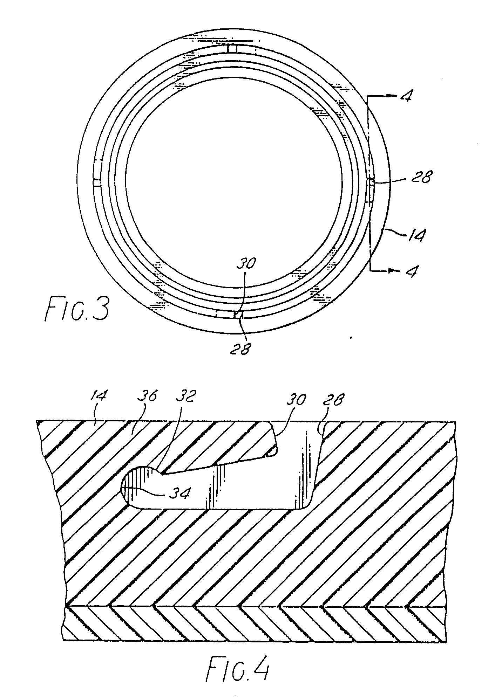

Referring to FIGS. 1, 3, and 4, bag side flange 14 has a plurality of tapered slots 28 having a wide entrance 30 to accept the pins 16 of the body side flange 12 and guide them to a locked position. A detent 32 adjacent to the terminal end 34 of the tapered slot 28 holds a pin 16 in the locked position. The arm 36 of the bag side flange 14 in which the tapered slots 28 are located is tapered axially on it's inside surface 38 to allow easy assembly with the body side flange 12 and to guide the parts 12, 14 to an interlocking seal as they are pressed together. The inside diameter of the bag side flange 14 has a short tapered arm 40 to hold the arm 42 of the body side flange 12 and the O-ring 18 in contact with the large arm 36 of the bag side flange. This contact area 38 at the inside diameter of the flanges also prevents solids from entering the seal area adjacent to O-ring 18. The inside diameter of the body side flange 12 is straight and smooth to facilitate easy cleaning.

All parts used in this flange concept have been designed for manufacturing by standard molding processes.

**Claims**

1. A coupling device for use in connecting an ostomy bag to a wearer, comprising a body side coupling flange having a substantially circular shape with an aperture therein adapted to surround a stomal opening in a wearer and a flat surface thereon adapted to interface with an adhesive material for connecting said body side coupling (12) to said wearer, and a bag side coupling flange (14) adapted to be connected to said body side coupling, said bag side coupling holding a bag for receiving material which passes from said wearer, said body side flange being connected to said bag side flange by the combination of a bayonet coupling with an O-ring seal (18) without requiring significant pressure to be applied against said body side flange; the O-ring seal providing a seal between said body side flange and said bag side flange, characterised in that the O-ring seal is comprised of an O-ring which is resiliently held within a radially outwardly opening annular groove (20) formed in an annular wall (42) of the body side flange; said body side flange further including a plurality of radially extending pins (16) and said bag side flange including a first annular wall (36) having a like number of openings (28) for receiving said pins, said openings having an L-shaped configuration whereby said bag side flange and body side flange can be joined and held together; the bag side flange also having a second annular wall (40) located radially inwardly of the first annular wall, said further annular wall having an outer surface which contacts (at 38) an inner surface of the body side flange annular wall (42) to prevent solids from entering the seal area adjacent to the O-ring (18).

**Revendications**

1. Dispositif d'accouplement servant à raccorder un sac de stomie à un utilisateur, comprenant une bride d'accouplement côté corps, de forme sensiblement circulaire, percée d'une ouverture conçue pour entourer une ouverture stomale (anus artificiel) d'un utilisateur et comportant une face plate conçue pour se coller sur une matière adhésive pour raccorder ledit accouplement (12) côté corps audit utilisateur, et une bride d'accouplement (14) côté sac conçue pour être raccordée audit accouplement côté corps, ledit accouplement côté sac portant un sac destiné à recevoir les matières qui arrivent dudit utilisateur, ladite bride côté corps étant raccordée à ladite bride côté sac par la combinaison d'un accouplement à baïonnette avec un joint d'étanchéité torique (18) sans qu'il soit nécessaire d'exercer une pression notable sur ladite bride côté corps; le joint d'étanchéité torique assurant l'étanchéité entre ladite bride côté corps et ladite bride côté sac, caractérisé en ce que le joint d'étanchéité torique se compose d'un joint torique qui est maintenu élastiquement à l'intérieur d'une gorge annulaire (20), s'ouvrant radialement vers l'extérieur, formée dans une paroi annulaire (42) de la bride côté corps; ladite bride côté corps comportant en outre plusieurs ergots (16) dirigés radialement et ladite bride côté sac comportant une première paroi annulaire (36) munie d'un même nombre d'ouvertures (28) pour recevoir lesdits ergots, lesdites ouvertures étant en forme de L pour que ladite bride côté sac et ladite bride côté corps puissent être réunies et maintenues assemblées; la bride côté sac ayant aussi une seconde paroi annulaire (40) située radialement à l'intérieur de la première paroi annulaire, ladite seconde paroi annulaire ayant une face extérieure qui touche (en 38) une face intérieure de la paroi annulaire (42) de la bride côté corps pour empêcher les matières solides de pénétrer dans la zone d'étanchéité contiguë au joint torique (18).

**Patentansprüche**

1. Kupplung für die Verbindung eines Ostomiebeutels an einem Träger, die aufweist: einen körperseitigen Kupplungsflansch von im wesentlichen kreisförmiger Form mit einer Öffnung darin, um eine Stomaöffnung in einem Träger zu umgeben, und einer flachen Oberfläche, die mit einem Haftmaterial verbunden werden kann, um die körperseitige Kupplung (12) mit dem Träger zu verbinden, und einen beutelseitigen Kupplungsflansch (14), der mit der körperseitigen Kupplung verbunden wird, wobei die beutelseitige Kupplung einen Beutel zum Aufnehmen von Material hält, das von dem Träger kommt, und der körperseitige Flansch mit dem beutelseitigen Flansch durch die Kombination einer Bajonettkupplung mit einer O-Ringdichtung (18) verbunden wird, ohne daß es erforderlich ist, einen bedeutenden Druck auf den körperseitigen Flansch auszuüben, und die O-Ringdichtung eine Dichtung zwischen dem körperseitigen Flansch und dem beutelseitigen Flansch bildet, **dadurch gekennzeichnet,** daß die O-Ringdichtung aus einem O-Ring besteht, der elastisch in einer sich radial nach außen öffnenden Ringnut (20) gehalten wird, die in einer ringförmigen Wand (42) des körperseitigen Flansches ausgebildet ist; wobei der körperseitige Flansch ferner mehrere sich radial erstreckende Stifte (16) und der beutelseitige Flansch eine erste ringförmige Wand (36) mit einer gleichen Zahl von Öffnungen (28) zur Aufnahme der Stifte aufweist, die Öffnungen eine L-förmige Gestalt haben und der beutelseitige Flansch und der körperseitige Flansch miteinander verbunden und zusammengehalten werden können; wobei der beutelseitige Flansch ferner eine zweite ringförmige Wand (40) aufweist, die radial nach innen von der ersten ringförmigen Wand angeordnet ist, und diese weitere ringförmige Wand eine äußere Oberfläche aufweist, die (bei 38) eine innere Oberfläche der ringförmigen Wand (42) des körperseitigen Flansches berührt, um zu verhindern, daß feste Stoffe in den Dichtungsbereich, angrenzend an den O-Ring (18), eintreten.

FIG.1

FIG.2

FIG.3

FIG.4